**Europäisches Patentamt**

**European Patent Office**

**Office européen des brevets**

(19)

(11) Veröffentlichungsnummer : **0 063 333**
**B1**

(12) **EUROPÄISCHE PATENTSCHRIFT**

(45) Veröffentlichungstag der Patentschrift :
30.12.86

(51) Int. Cl.⁴ : **A 61 M 25/00**, F 16 L 37/08

(21) Anmeldenummer : **82103061.6**

(22) Anmeldetag : **08.04.82**

(54) Anschlussvorrichtung für medizinische Instrumente oder Leitungen.

(30) Priorität : **13.04.81 DE 3114937**

(43) Veröffentlichungstag der Anmeldung :
**27.10.82 Patentblatt 82/43**

(45) Bekanntmachung des Hinweises auf die Patenterteilung : **30.12.86 Patentblatt 86/52**

(84) Benannte Vertragsstaaten :
**DE FR GB IT**

(56) Entgegenhaltungen :
**DE-A- 1 766 739**
**DE-A- 1 961 893**
**DE-A- 2 657 215**
**DE-A- 2 718 956**
**US-A- 3 768 476**

(73) Patentinhaber : **Fresenius AG**
**Gluckensteinweg 5**
**D-6380 Bad Homburg (DE)**

(72) Erfinder : **Krütten, Viktor**
**Buchenstrasse 6**
**D-6690 St.Wendel (DE)**

(74) Vertreter : **Luderschmidt, Wolfgang, Dr. Dipl-Chem.**
**Görtz, Dr. Fuchs, Dr. Luderschmidt Patentanwälte**
**Sonnenberger Strasse 100 Postfach 26 26**
**D-6200 Wiesbaden (DE)**

Jouve, 18, rue St-Denis, 75001 Paris, France

## Beschreibung

Die Erfindung betrifft eine Anschlußvorrichtung für medizinische Instrumente oder Leitungen wie Katheter, Sonden, Schläuche usw. insbesondere zur Injektion, Transfusion oder Infusion, nach dem Oberbegriff des Anspruchs 1.

Als derartige Anschlußvorrichtungen werden in der Praxis vor allem sogenannte Luer- oder Record-Verbindungen eingesetzt. Typischerweise ist dabei am einen Teil eine lose oder instrumentenfeste Überwurfmutter mit doppelgängigem Gewinde mit einer Gewindesteigung von in der Regel 12° vorgesehen, welche das Aufnahmeteil bildet. Im Innenraum des Gewindes ist ein Kegelschaft mit innerem Durchflußkanal angeordnet. Das Einsteckteil ist als Kegelhülse mit entsprechend kegeliger Innenoberfläche ausgeführt, die den Außenkegel des Kegelschaftes übergreift und an diesem dicht anliegt, wenn das Einsteckteil mit seiner Umfangswand in den Raum zwischen Gewinde und Kegelschaft eingeführt wird. Am Außenumfang weist das Einsteckteil als Gewindeabschnitte ausgebildete Haltevorsprünge auf, die in das doppelgängige Innengewinde der Überwurfmutter eingreifen, so daß durch gegenseitige Verdrehung von Aufnahmeteil und Einsteckteil eine Einschraubbewegung erfolgt, bis der Kegelschaft sauber in der Kegelhülse sitzt. Eine Ausbildung beider aneinander anliegender Oberflächen von Schaft und Hülse in Kegel- oder Konusform ist jedoch nicht für alle Verbindungen erforderlich ; so kann nur eine der Oberflächen kegelig oder konisch ausgebildet sein, so daß sich eine mehr linienförmige gegenseitige Anlage, die aber immer noch dicht ist, ergibt, oder es können die Überwurfmutter mit Zylinderschaft und das Einsteckteil als Zylinderhülse ausgebildet werden, wenn die gegenseitige Endposition etwa durch radiale Anschläge gesichert wird, oder auf Dichtheit kein Wert gelegt wird.

Zur Herstellung des Anschlusses wird das Einsteckteil mit den Haltevorsprüngen an das Innengewinde des Aufnahmeteils angesetzt, und sodann das Einsteckteil in das Aufnahmeteil eingeschraubt, bis durch die damit erzeugte axiale Relativverschiebung der Hülse des Einsteckteiles auf dem Schaft des Aufnahmeteiles die gewünschte Endstellung erreicht ist. Im Falle einer Kegelverbindung ergibt sich eine ausreichende Haftreibung der unter Druck aneinanderliegenden Kegelflächen, welche diese gegenseitige Verbindung sichert. Die Überwurfmutter sichert die Verbindung zusätzlich durch den Eingriff der Haltevorsprünge in die Gewindegänge der Überwurfmutter, so daß die Verbindung auch gegenüber etwa auftretenden starken Zug- oder Biegebelastungen gesichert ist.

Insbesondere bei einer Ausbildung des Einsteck- und Aufnahmeteiles aus Kunststoff (US-A-3 768 476), was schon aus Kostengründen für derartige, in der Regel nicht wiederverwendete Anschlußvorrichtungen bevorzugt ist, ergibt sich in der Endstellung der Überwurfmutter am Gewindeabschnitt trotz der relativ hohen Steigung des Gewindes eine selbsthemmende Wirkung dadurch, daß die aneinander anliegenden Gewindeteile unter geringfügiger elastischer Verformung aneinander anliegen und durch Haftreibung in dieser Stellung gehalten sind ; dies setzt allerdings eine ausreichende Kraftanwendung beim Aufschrauben der Überwurfmutter voraus. Wenn jedoch die Überwurfmutter mit zu geringer Kraft aufgeschraubt ist oder etwa der Patient an der Überwurfmutter dreht, oder eine Lockerung der Überwurfmutter aus der aufgeschraubten Endstellung aus irgendeinem sonstigen Grund erfolgt, so entfällt auch bei Ausführung aus Kunststoff diese Lagesicherung der Überwurfmutter. Hierzu ist nur eine ganz geringe Drehung der Überwurfmutter in Löserichtung erforderlich, die bei optischer Überprüfung der Verbindung nicht wahrgenommen wird. Dennoch aber wirkt die Überwurfmutter bereits in einer solchen geringfügig gelockerten Stellung nicht mehr an der formschlüssigen Lagesicherung zwischen Einsteckteil und Aufnahmeteil mit, sondern werden Schaft und Hülse nur noch durch Haftreibung aneinander gehalten, sofern überhaupt eine Kegelverbindung vorgesehen ist. Bei entsprechender Zug- oder Biegebelastung, wie sie etwa bei unsorgfältigen Handhabungen häufig auftreten können, löst sich diese Haftverbindung, so daß zumindest die Dichtheit der Verbindung aufgehoben ist, wenn nicht das Einsteckteil aus dem Aufnahmeteil völlig herausfällt. Es liegt auf der Hand, daß dies äußerst weitreichende, sogar tödliche Folgen haben kann.

Um derartige Risiken eines unbeabsichtigten Lösens der Anschlußvorrichtung zu vermeiden, ist aus der DE-A-27 18 956, von der die Erfindung ausgeht, bereits eine unlösbare Anschlußvorrichtung in Form einer Schlauchkupplung bekannt geworden. Diese bekannte Anschlußvorrichtung besteht im wesentlichen aus einem Einsteckteil mit im Querschnitt quadratischem Einsteckabschnitt und einem entsprechend geformten Aufnahmeteil. Im Bereich des Einsteckabschnitts stehen an den vier Ecken in Einsteckrichtung vier federnde Rastfinger ab, die nach außen gerichtete Haltevorsprünge tragen. Das Aufnahmeteil ist mit einer quadratischen axialen Einstecköffnung zur Aufnahme des Einsteckabschnittes des Einschnitteils versehen. Von der Einstecköffnung erstrecken sich an deren vier Ecken Führungskanäle axial in Einsteckrichtung nach hinten, die zur Aufnahme der Rastfinger dienen. In den Führungskanälen sind jeweils Rastvorsprünge angebracht. Beim Einführen des Einsteckteiles in das Aufnahmeteil werden die federnden Rastfinger im Bereich der Rastvorsprünge gebogen ; beim weiteren Einführen gelangen die Haltevorsprünge der Rastfinger über die Rastvorsprünge in die Führungskanäle, so daß dadurch die Verbindung verriegelt wird. Die Dichtflächen stehen dabei senkrecht zur Einsteckrichtung und liegen plan aufeinander. Für eine verbesserte Abdichtung werden in Aufnahmeprofile Dichtringe eingelegt. Da die Dichtflächen gegen Berührung nicht sonderlich geschützt sind, werden diese durch aufgeklebte Streifen abgedeckt, die nach Herstellung einer sterilen Verbindung durch einen Schlitz in der Kupplung herausziehbar sind.

Mit genormten Luer-Kupplungsteilen kann keines der beiden Anschlußteile dieser bekannten Anschlußvorrichtung zusammenwirken. Luer-Kupplungsteile enthalten eine genormte Kegeldichtung, die mit der bekannten Anschlußvorrichtung nicht zusammenpaßt. Die Haltevorsprünge weiblicher Luer-Einsteckteile sind nicht elastisch i. S. des bekannten Einsteckteiles ausgebildet und stehen nicht als federnde Rastfinger ab. Ein Zusammenwirken der bekannten Anschlußvorrichtung mit Luer-Kupplungsteilen ist daher auch bei einer räumlichen Modifikation und Anpassung an dessen Abmessungen ausgeschlossen. Bei Verwendung der bekannten Anschlußvorrichtung müßten daher entweder die derzeit benutzten Instrumente an den Anschlußteilen entsprechend umgerüstet werden, oder aufwendige Adapterteile benutzt werden. Als Problemstellen bezüglich Sterilität und Dichtheit sind bei medizinischen Leitungen meist nur Anschlußstellen und Kupplung anzusehen. Zusätzliche Adapterteile, die Fehlbedienungen und Fehlerquellen erhöhen, sind daher abgesehen von dem erforderlichen Zusatzaufwand auch unter diesem Gesichtspunkt unerwünscht und möglichst zu vermeiden.

Bei der Anschlußvorrichtung gemäß der DE-A-27 18 956 ist weiterhin nachteilig, daß keine saubere Gassterilisation durchgeführt werden kann. Bei der üblichen Gassterilisation von medizinischen Anschlußvorrichtungen werden die Einzelteile in einem Gasstrom sterilisiert. Die Einzelteile dürfen daher keine geschlossenen Hohlräume enthalten, die vom Gas nicht durchströmt werden können. Bei der bekannten Anschlußvorrichtung werden hinter den eingelegten Dichtringen solche zu vermeidenden Räume geschaffen, so daß die Sterilisation unbefriedigend ist oder durch aufwendige Maßnahmen verteuert wird. Weiterhin besteht die Gefahr, daß die federnden, relativ lang und dünn ausgebildeten Rastfinger beim ungeschickten Einführen abbrechen oder sich verklemmen, so daß die Verbindung nicht geschlossen oder zumindest undicht ist. Diese Gefahr besteht um so mehr, als keine optische Kontrollmöglichkeit für die sichere Einrastung gegeben ist.

Aufgabe der Erfindung ist es demgegenüber, eine Anschlußvorrichtung der im Oberbegriff des Anspruchs 1 angegebenen Gattung zu schaffen, bei der das Einsteckteil nicht nur mit dem Aufnahmeteil eine unlösbare Steckverbindung bilden, sondern auch mit männlichen Luer-Kupplungsteilen eine lösbare Verbindung bilden kann bzw. umgekehrt das Aufnahmeteil nicht nur ein speziell angepaßtes Einsteckteil der Anschlußvorrichtung, sondern auch ein genormtes weibliches Luer-Kupplungsteil zur Bildung einer unlösbaren Steckverbindung aufnehmen kann.

Diese Aufgabe wird durch die kennzeichnenden Merkmale des Anspruches 1 gelöst.

Danach ist in der Einsteköffnung des Aufnahmeteiles im axialen Bereich des Führungsabschnittes ein Einsteckschaft koaxial angeordnet. Dieser Einsteckschaft ist konisch ausgeführt und kann zur Herstellung einer dichten Verbindung in ein weibliches Luer-Kupplungsteil eingeführt werden. Zwischen dem Einsteckschaft und der Innenseite der Umfangswand des Führungsabschnittes ist ein ringförmiger Zwischenraum gebildet, in dem in Führungskanälen, die die Außengewindeelemente bildenden Haltevorsprünge eines weiblichen Luer-Kupplungsteiles zum Zusammenstecken geführt werden können. Die Umfangswand ist elastisch ausgebildet und enthält im Endbereich der Führungskanäle Rastvorsprünge. Beim Zusammenstecken der Anschlußvorrichtung wird die Umfangswand elastisch aufgeweitet und die Haltevorsprünge können so hinter die Rastvorsprünge geführt werden, so daß eine feste unlösbare Verbindung entsteht. Durch die ringförmige Umfangswand um den Einsteckschaft ist auch gewährleistet, daß der Einsteckschaft beim Hantieren nicht berührt wird und so die Sterilisation an den dichtenden Flächen erhalten bleibt.

Somit wird eine sichere und unlösbare Steckverbindung mit einer bewährten Dichtungstechnik geschaffen, wobei der Aufnahmeteil bei Bedarf ein genormtes weibliches Luer-Kupplungsteil als Einsteckteil verriegeln kann, das zuvor zur Bildung anderer, lösbarer Verbindungen bei Bedarf tatsächlich als Luer-Kupplungsteil verwendet worden sein kann. Probleme bei der Gassterilisation treten nicht auf, da keine geschlossenen oder für einen Gasstrom schlecht zugängliche Kammern oder Hinterschneidungen in den Einzelteilen enthalten sind. Da abstehende, verformungs- und bruchgefährdete Teile vollständig vermieden sind, besteht keine Gefahr von Beschädigungen oder unvollständig eingerasteten Endlagen.

Aus der DE-A-26 57 215 ist es zwar bekannt, ein Einsteckteil in einem Einsteckabschnitt von im wesentlichen kreisförmigem Querschnitt zu verwenden, das mit einem umfangsseitigen Haltevorsprung einen elastischen Rastvorsprung des Aufnahmeteils hintergreift. Hier handelt es sich jedoch um eine schnellösbare Steckverbindung, bei der der Haltevorsprung als umlaufender Wulst in einem in Einsteckrichtung hinteren Bereich des Einsteckteils ausgebildet ist und von zwei einander diametral gegenüberliegenden Rastvorsprüngen am Vorderende des Aufnahmeteils hintergriffen wird, die durch Verformung des Vorderendes des Aufnahmeteiles außer Eingriff mit dem Haltevorsprung gebracht werden können, um so die Kupplung bei Bedarf schnell wieder lösen zu können. Eine Kompatibilität mit Luer-Kupplungsteilen ist somit nicht erzielbar.

Da neben besonders angepaßten Einsteckteilen für eine erfindungsgemäße Anschlußvorrichtung auch mit genormten weiblichen Luer-Kupplungs-

teilen mit Außengewindezapfen gearbeitet werden kann, wird die Erfindung in ihrer einfachsten Form bereits dadurch nutzbar, daß lediglich entsprechend ausgebildete Aufnahmeteile zur Verfügung gestellt werden.

Die Unteransprüche haben vorteilhafte Weiterbildungen der Erfindung zum Inhalt.

Die Erfindung wird nachstehend anhand eines Ausführungsbeispiels näher erläutert.

Es zeigt

Figur 1 ein Aufnahmeteil einer erfindungsgemäßen Anschlußvorrichtung in Seitenansicht,

Figur 2 ein zugehöriges Einsteckteil in Seitenansicht,

Figur 3 eine Draufsicht auf das Aufnahmeteil gemäß Fig. 1 aus Richtung des Pfeiles III in Fig. 1,

Figur 4 eine Ansicht des Einsteckteiles gemäß Fig. 2 aus Richtung des Pfeiles IV in Fig. 2 und

Figur 5 einen Schnitt durch das Aufnahmeteil gemäß den Fig. 1 und 3 gemäß Linie V-V in Fig. 3, wobei das angeschlossene Einsteckteil strichpunktiert in seiner Relativlage zum Aufnahmeteil angedeutet ist.

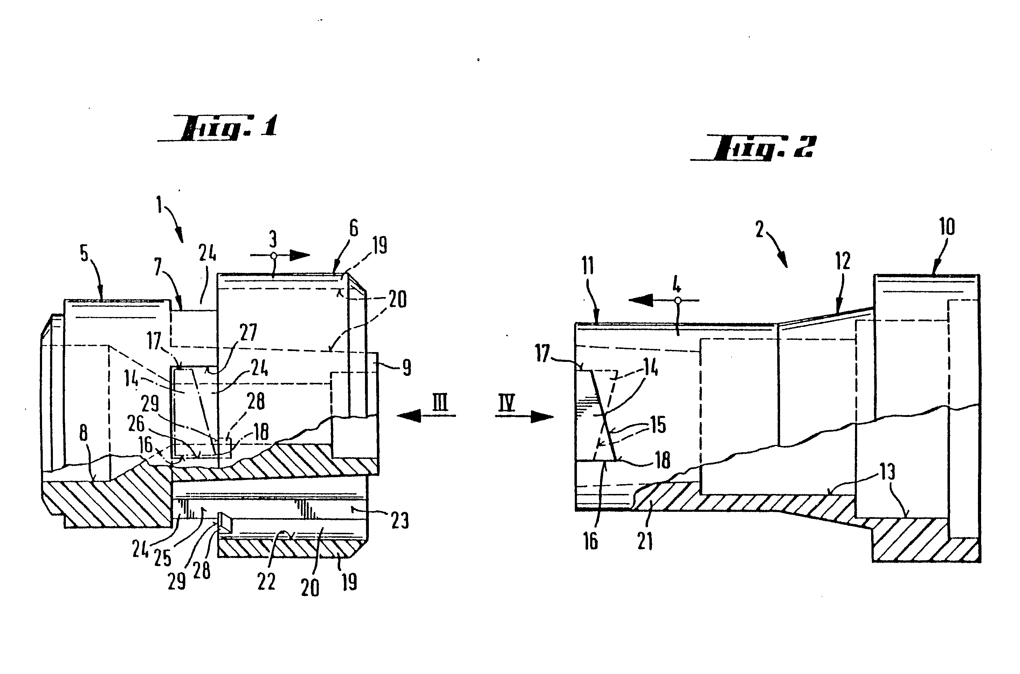

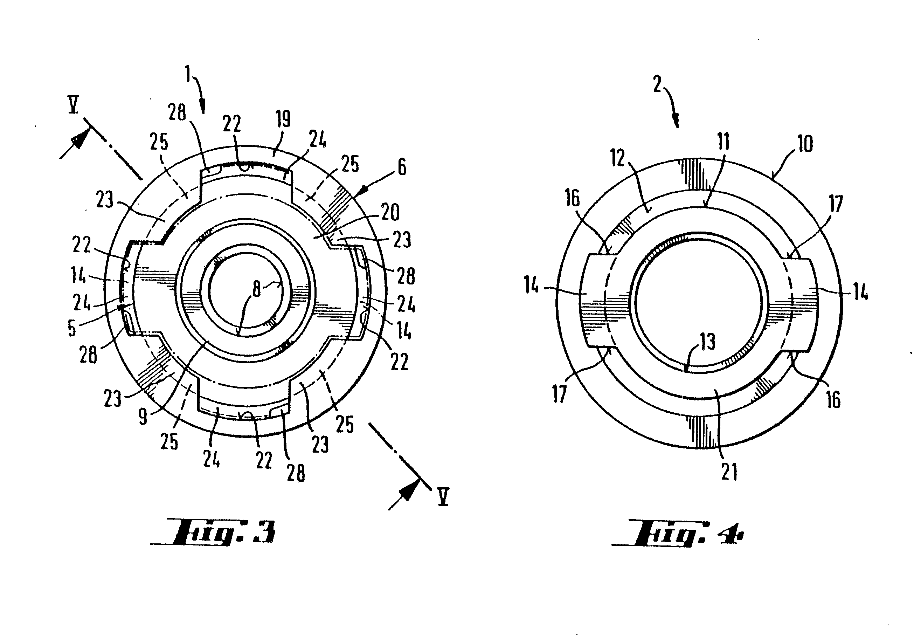

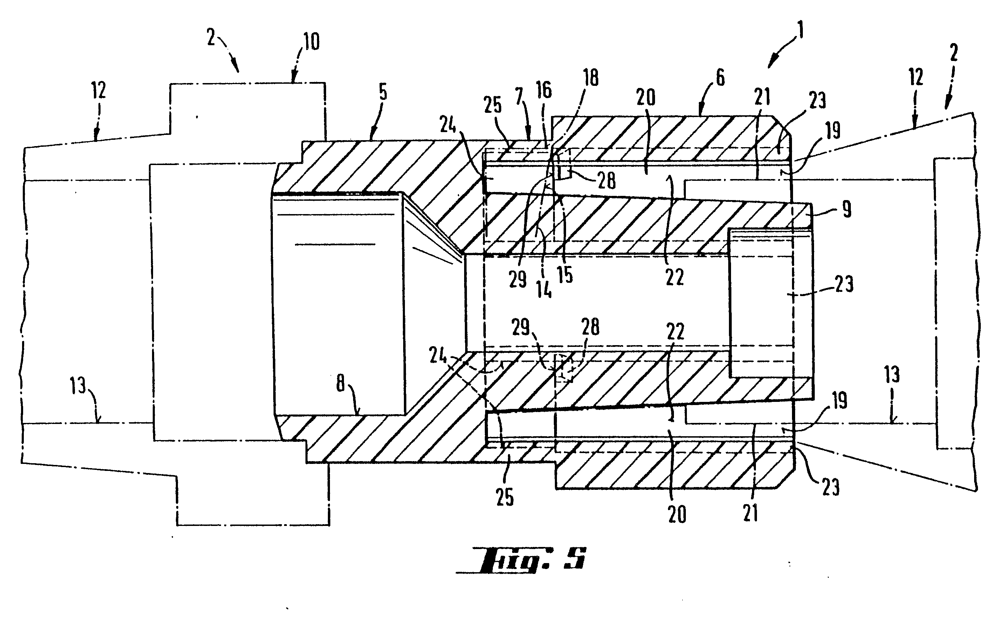

In Fig. 1 ist mit 1 ein Aufnahmeteil und in Fig. 2 mit 2 ein Einsteckteil veranschaulicht, die gemäß Pfeilen 3 bzw. 4 axial gegeneinander geführt eine erfindungsgemäße Anschlußvorrichtung ergeben. Das Aufnahmeteil 1, welches in Fig. 5 vergrößert in einem Schnitt gemäß Linie V-V in Fig. 3 dargestellt ist und ein selbständig handelbares Teil darstellt, weist einen Sockelabschnitt 5, einen Führungsabschnitt 6 und dazwischen einen Halteabschnitt 7 auf. Am Sockelabschnitt 5 kann im Beispielsfalle etwa ein nicht näher dargestellter Schlauch derart befestigt werden, daß sein Innenraum mit einem Durchtrittskanal 8 in Verbindung steht, der koaxial im Sockelabschnitt 5 herausgearbeitet ist und im Bereich des Halteabschnitts 7 und des Führungsabschnitts 6 in einem den Führungsabschnitt 6 koaxial durchsetzenden Einsteckschaft 9 des Aufnahmeteils 1 geführt ist.

Das im Querschnitt wesentliche runde Einsteckteil 2 weist ebenfalls einen Sockelabschnitt 10, einen Einsteckabschnitt 11 und einen dazwischen angeordneten Übergangsabschnitt 12 auf. Das Einsteckteil 2 ist insgesamt etwa hülsenförmig ausgebildet und besitzt im Beispielsfalle einen inneren Durchtrittskanal 13, der jedenfalls im Bereich des Einsteckabschnitts 11 eine lichte Weite besitzt, die ein Übergreifen des Einsteckschaftes 9 des Aufnahmeteils 1 ermöglicht. Die Außenoberfläche des Einsteckschaftes 9 und/oder die Wandfläche des Durchtrittskanals 13 im Bereich des Einsteckabschnittes 1 können in an sich bekannter Weise konisch oder kegelförmig mit einer Steigung von 1 : 10 oder 1 : 16,67 wie dies im ersteren Fall für Record-Verbindungen und im zweiten Fall für Luer-Verbindungen üblich ist, ausgeführt sein, um eine dichte Preßanlage zwischen diesen Flächen zu ermöglichen. Jedoch kann auch die Außenoberfläche des Einsteckschaftes 9 und die Innenwand des Durchtrittskanals 13 zylinderförmig ausgeführt werden, derart, daß der Durchtrittskanal 13 auf der Oberfläche des Einsteckschaftes 9 ohne Klemmung, jedoch mit möglichst geringem Spiel, gleiten kann. Auch dann erfolgt eine radiale Führung des Einsteckabschnitts 11 des Einsteckteiles 2 am Außenumfang des Einsteckschaftes 9, wobei der Durchtrittskanal 13 im Einsteckteil 2 außerhalb des Einsteckabschnitts 11 bzw. der axialen Erstreckung des Einsteckschaftes 9 auch geschlossen werden könnte, wenn keine Strömungsverbindung erforderlich ist. Im Beispielsfalle möge die Anschlußvorrichtung zur Leitungsverbindung eines strömenden Mediums dienen, und ist die Außenumfangsfläche des Einsteckschaftes 9 in Richtung auf das Vorderende des Aufnahmeteils 1 konisch oder kegelförmig verjüngt mit einer Steigung von 1 : 16,67 ausgebildet, wie dies für Luer-Verbindungen üblich ist. Der Durchtrittskanal 13 des Einsteckteiles 2 ist hingegen im Bereich des Einsteckabschnitts 11 zylinderförmig ausgeführt und übergreift den Vorderteil des Einsteckschaftes 9 mit entsprechendem Spiel, während im rückwärtigen Teil ein entsprechender dichter Preßsitz geschaffen wird. Statt dessen könnte auch die Umfangswand des Durchtrittskanals 13 im Einsteckabschnitt 11 des Einsteckteiles 2 konisch oder kegelförmig zum Vorderende des Einsteckteiles 2 sich erweiternd ausgebildet werden und so im wesentlichen flächig an dem gleichsinnig verjüngten Umfang des Einsteckschaftes 9 anliegen. Als Vorderende des Aufnahmeteils 1 und des Einsteckteils 2 im Rahmen der vorliegenden Anmeldung ist jeweils das dem jeweiligen Gegenteil zugewandte Ende zu verstehen, so daß der Pfeil 3 bezüglich des Aufnahmeteils 1 und der Pfeil 4 bezüglich des Einsteckteiles 2 jeweils die Richtung « vorne » angibt.

Am vorderen Ende des Einsteckabschnitts 11 des Einsteckteiles 2 sind, wie auch Fig. 4 veranschaulicht, zwei einander diametral gegenüberliegend am Außenumfang des Einsteckabschnitts 11 angeordnete Haltevorsprünge 14 befestigt. Die Haltevorsprünge 14 weisen im Beispielsfalle Rückflächen 15 (vgl. Fig. 2) auf, die eine Neigung gegenüber einer Radialebene von z. B. 12° besitzen, wie dies der Gewindesteigung einer üblichen Überwurfmutter einer Luer-Verbindung entspricht. Damit werden optimale Eingriffsverhältnisse erzeugt, wenn das Einsteckteil 2 mit seinem Einsteckabschnitt 11 in die Überwurfmutter einer Luer-Verbindung eingeschraubt wird. Ein Einsteckteil 2 der veranschaulichten Ausbildung ist sogar für eine solche Luer-Verbindung in Gebrauch, so daß für das Einsteckteil 2 einer erfindungsgemäßen Anschlußvorrichtung bei Bedarf auf das bereits im Handel befindliche Einsteckteil für diese Luer-Verbindung zurückgegriffen werden kann.

Im Beispielsfalle besitzt jeder Haltevorsprung 14 eine Umfangserstreckung von etwas weniger als 90° und in Umfangsrichtung seitliche Anschlagenden 16 und 17 sowie an der in Einsteckrichtung gemäß Pfeil 4 hinteren Seite des Haltevorsprungs 14 einen Anschlag 18, der im Beispielsfalle durch die Kante gebildet ist, die sich zwischen der das Anschlagende 16 bildenden Seitenfläche und der geneigten Rückfläche 15 des Haltevorsprungs 14 ergibt. Die Funktion der Anschlagenden 16 und 17 sowie des Anschlags 18 wird weiter unten noch näher erläutert.

Zwischen der dünn gehaltenen Umfangswand 19 des Führungsabschnitts 6 und dem Außenumfang des Einsteckschaftes 9 ist ein ringkanalförmiger Zwischenraum 20 vorgesehen, in den die mit 21 bezeichnete Umfangswand des Einsteckabschnitts 11 des Einsteckteiles 2 samt Haltevorsprüngen 14 einschiebbar ist, bis die Haltevorsprünge 14 an die Rückseite des Führungsabschnittes 6 und in den Halteabschnitt 7 des Aufnahmeteils 1 gelangen. Wie insbesondere aus den Fig. 3 und 5 ersichtlich ist, weist der Führungsabschnitt 6 an seiner Innenseite vier im Winkel von 90° zueinander liegende Führungskanäle 22 zwischen axialen Führungsrippen 23 auf, in denen die Haltevorsprünge 14 axial sauber geführt sind, wie dies in Fig. 3 strichpunktiert veranschaulicht ist.

Der Halteabschnitt 7 des Aufnahmeteiles 1 weist fluchtend mit den Führungskanälen 22 Ausnehmungen 24 auf, die am Umfang der Wand des Halteabschnitts 7 zwischen Rippen 25 angeordnet sind, welche wiederum die Verlängerung der Führungsrippen 23 darstellen. Die Ausbildung der Ausnehmungen 24 als radiale Durchbrüche ist nicht zwingend erforderlich, jedoch fertigungstechnisch einfach und ermöglicht die optische Überprüfung des richtigen Sitzes der Haltevorsprünge 14 in den Ausnehmungen 24. Die Ausnehmungen 24 weisen zwei einander gegenüberliegende Gegen-Anschlagenden 26 und 27 für die Anschlagenden 16 bzw. 17 eines eingeführten Haltevorsprunges 14 auf, derart, daß ein eingeführter Haltevorsprung 14 in jeder Ausnehmung 24 gegen Verdrehen gesichert ist. Da die Gegen-Anschlagenden 26 und 27 in sehr geringem Abstand von den Anschlagenden 16 und 17 eines eingeführten Haltevorsprungs 14 liegen, kann eine fast spielfreie Verdrehsicherung problemlos erzielt werden. In jedem Führungskanal 22 ist, im Beispielsfalle in der Nachbarschaft der Führungsrippe 23, ein Rastvorsprung 28 angeordnet, der in den Bewegungsweg eines eingeführten Haltevorsprunges 14 ragt. Die Rastvorsprünge 28 können an ihrer in Einsteckrichtung gemäß Pfeil 3 vorderen Seite eine Auflaufschräge besitzen, wie dies im geschnittenen Bereich der Fig. 1 veranschaulicht ist, so daß die radiale Außenkante eines eingeführten Haltevorsprungs 14 weich auflaufen kann und den Rastvorsprung 28 unter Verformung der entsprechend dünnen Umfangswand 19 des Führungsabschnitts 6 sowie gegebenenfalls auch unter Verformung des Materials des Rastvorsprungs 28 radial nach außen drücken kann. Je nach den gewählten Materialstärken kann dabei gleichzeitig unter entsprechender geringfügiger Verformung der Umfangswand 21 des Einsteckabschnittes 11 des Einsteckteiles 2 auch ein Einfedern jedes Haltevorsprungs 14 radial nach innen erfolgen. Wenn der Haltevorsprung 14 unter elastischer Verformung am Rastvorsprung 28 vorbeigetreten ist, federt dieser und/oder der Haltevorsprung 14 wieder in die ursprüngliche Radiallage zurück, und bietet der Rastvorsprung 28 an seiner in Einsteckrichtung gemäß Pfeil 3 hinteren Seite eine Anschlagfläche 29, die im Beispielsfalle in einer Radialebene liegt und unmittelbar an die zugeordnete Ausnehmung 24 anschließt und in der Ebene der Rückfläche des Führungsabschnittes 6 liegt. Wenn nun versucht wird, das Einsteckteil 2 aus dem Aufnahmeteil 1 entgegen der Einsteckrichtung gemäß Pfeil 4 herauszuziehen, so gelangt der Anschlag 18 des Haltevorsprungs 14 zur Anlage an die zurückgefederte Anschlagfläche 29 des zugehörigen Rastvorsprungs 28. Dadurch ist jeder Haltevorsprung 14 in Einsteckrichtung gemäß Pfeil 3 hinter dem Rastvorsprung 28 verriegelt und, bei entsprechender radialer Ausbildung des Anschlags 18 und der Anschlagfläche 29, das Einsteckteil 2 im normalen Gebrauch absolut unlösbar im Aufnahmeteil 1 gehalten. Wenn, wie im Beispielsfalle, wenigstens zwei einander gegenüberliegende Haltevorsprünge 14, oder aber bei Bedarf auch vier entsprechend den Führungskanälen 22 angeordnete Haltevorsprünge 14 vorgesehen sind, die in gleicher Axiallage am Einsteckabschnitt 11 des Einsteckteiles 2 angeordnet sind, so ergibt sich eine symmetrische Abstützung des Einsteckteiles 2 an den Anschlägen 18, und damit eine saubere Lagerung und Lagesicherung.

Die Sockelabschnitte 5 und 10 des Aufnahmeteiles 1 bzw. des Einsteckteiles 2 sind in ihrer Ausbildung derart aufeinander abgestimmt, daß der Sockelabschnitt 10 des Einsteckteiles 2 auf den Sockelabschnitt 5 des Aufnahmeteiles 1 aufgesetzt und dort beispielsweise verklebt werden kann, wie dies in Fig. 5 strichpunktiert veranschaulicht ist, um ein Anschlußelement zu erhalten, welches einenends zur Aufnahme eines weiteren Einsteckteiles 2 dienen kann, wie dies in Fig. 5 ebenfalls strichpunktiert veranschaulicht ist, und andernends in ein Aufnahmeteil 1 einsteckbar ist. Entsprechend können Endabschnitte von Leitungen od. dgl. mit Anschlußarmaturen versehen werden, welche in ihrer Ausbildung den Sockelabschnitten 5 und 10 entsprechen und so am jeweils andersartigen Sockelabschnitt 10 oder 5 etwa durch Klebung befestigt werden, wonach die Leitungsenden mit je einem Aufnahmeteil 1 einerseits und einem Einsteckteil 2 andererseits versehen und bereit zu einer Verbindung mittels der erfindungsgemäßen Anschlußvorrichtung sind. Selbstverständlich kann aber auch anstelle der Sockelabschnitte 5 oder 10 eine völlig andere, dem jeweiligen Zweck entsprechende Ausbildung der jeweils funktionell für die Herstellung der eigentlichen Verbindung nicht benötigten Teile des Aufnahmeteils 1 und des Sockelteiles 2 erfolgen, je nach Bedarf im Einzelfall.

Grundsätzlich ist die erfindungsgemäße Anschlußvorrichtung stets da verwendbar, wo bisher in der Praxis sogenannte Luer- oder Record-Verbindungen verwendet wurden. Dabei kann in der geschilderten Weise eine dichte oder auch nicht dichte Verbindung zwischen dem Aufnahmeteil 1 und dem Einsteckteil 2 durch einfaches Einstecken und Einrasten geschaffen werden. Wesentlich ist dabei, daß die Haltevorsprünge 14 im Bereich des Vorderendes des Einsteckabschnittes 11 des Einsteckteiles 2 durch den Führungsabschnitt 6 hindurchgeführt und hinter diesem an den Rastvorsprüngen 28 verrastet werden, während der Führungsabschnitt 6 mit den Führungsrippen 23 den Außenumfang des Einsteckabschnittes 11 mit geringem Spiel umgibt und so führt. Bei entsprechender Ausbildung des rückwärtigen Anschlags 18 der Haltevorsprünge 14 einerseits und der Anschlagfläche 29 der Rastvorsprünge 28 andererseits kann dabei ohne jegliche Drehbewegung eine absolut unverlierbare und unlösbare Halterung des Einsteckteiles 2 im Aufnahmeteil 1 erreicht werden, die nur unter Zerstörung der Teile wieder gelöst werden kann und infolge der sauberen gegenseitigen Führung von Aufnahmeteil 1 und Einsteckteil 2 hohen mechanischen Belastungen praktisch verformungsfrei standhält. Wenn sowohl die Außenumfangsfläche des Einsteckschaftes 9 als auch die Durchtrittsöffnung 13 im Bereich des Einsteckabschnittes 11 des Einsteckteiles 2 beide zylindrisch oder beide konisch oder kegelförmig ausgebildet sind, ergibt sich in diesem Bereich eine zusätzliche weitere Abstützung von Aufnahmeteil 1 und Einsteckteil 2 gegeneinander, sowie bei Bedarf eine fluiddichte Verbindung zwischen den Durchtrittskanälen 8 und 13. Das Einsteckteil 2 kann bei Bedarf nacheinander an eine beliebige Anzahl von weiblichen Luer-Verbindungsteilen mit Überwurfmutter angeschlossen und schließlich zur unlösbaren endgültigen Verbindung in ein erfindungsgemäßes Aufnahmeteil 1 eingeschoben werden.

## Patentansprüche

1. Anschlußvorrichtung für medizinische Instrumente oder Leitungen wie Katheter, Sonden, Schläuche usw. insbesondere zur Injektion, Transfusion oder Infusion mit einem einen Durchtrittskanal (13) aufweisenden Einsteckteil (2) mit einem Einsteckabschnitt (11) mit wenigstens einem radialen Haltevorsprung (14) auf seiner Umfangswand (19), und mit einem einen Durchtrittskanal (8) aufweisenden Aufnahmeteil (1) mit einer zur Aufnahme des Einsteckabschnittes (11) des Einsteckteils (2) vorgesehenen mittleren axialen Einsteköffnung mit wenigstens einem Rastvorsprung (28) im Bewegungsweg des Haltevorsprunges (14) bei der Kupplungsbewegung zwischen dem Einsteckteil (2) und dem Aufnahmeteil (1), der unter elastischer Verformung einen Durchtritt des Haltevorsprungs (14) gestattet und an seiner in Durchtrittsrichtung hinteren Seite eine Anschlagfläche (29) zur Beaufschlagung durch einen in Durchtrittsrichtung hinteren Anschlag des durchgetretenen Haltevorsprungs (14) aufweist, wobei der Haltevorsprung (14) im Bereich des Vorderendes des Einsteckabschnittes (11) des Einsteckteiles (2) angeordnet und in Umfangsrichtung auf einen Winkelbereich von weniger als 180°, insbesondere weniger als 90°, begrenzt ist, und wobei das Aufnahmeteil (1) zwischen dem Rastvorsprung (28) und seinem Vorderende einen die Einsteköffnung umfangsseitig begrenzenden Führungsabschnitt (6) und wenigstens einen axialen Führungskanal (22) für den wenigstens einen Haltevorsprung (14) an der Innenseite der zylindrischen Umfangswand (19) des Führungsabschnitts (6) aufweist, wobei im hinteren Ende des Führungskanals (22) der Rastvorsprung (28) ausgebildet ist, dadurch gekennzeichnet,

a) daß das Einsteckteil (2) einen im Querschnitt im wesentlichen runden Einsteckabschnitt (11) aufweist,

b) daß in der Einsteköffnung des Aufnahmeteiles (1) im axialen Bereich des Führungsabschnittes (6) ein Einsteckschaft (9) koaxial angeordnet ist, dessen Umfangsoberfläche unter Bildung eines ringkanalförmigen Zwischenraums (20) im Abstand von der Innenseite der Umfangswand (19) des Führungsabschnittes (6) liegt, und daß der Einsteckschaft (9) einen inneren Durchtrittskanal (8) enthält,

c) daß die Umfangsoberfläche des Einsteckschaftes (9) zu dessen Vorderende hin im Kegelverhältnis 1 : 16,67 gemäß einer Luer-Verbindung konisch verjüngt und die Innenoberfläche des Durchtrittskanals (13) des Einsteckteiles (2) zu dessen Vorderende hin konisch erweitert ist,

d) daß das elastische Element, das den Durchtritt des im wesentlichen unelastischen Haltevorsprungs (14) gestattet, die Umfangswand (19) des Führungsabschnittes (6) ist, und

e) daß jeder Haltevorsprung (14) eine keilförmig abgeschrägte, in Einsteckrichtung (Pfeil 4) hintere Rückfläche (15) aufweist und die Neigung der Rückfläche (15) wenigstens annähernd der Gewindesteigung einer gebräuchlichen Überwurfmutter für Luer-Verbindungen (normalerweise 12°) entspricht.

2. Anschlußvorrichtung nach Anspruch 1, dadurch gekennzeichnet, daß das Einsteckteil (2) wenigstens zwei Haltevorsprünge (14) aufweist, daß jeder Haltevorsprung (14) des Einsteckteiles (2) einem anderen Haltevorsprung (14) in paarweiser Zuordnung diametral gegenüberliegt, und daß zwischen den Führungskanälen (22) für die Haltevorsprünge (14) Führungsrippen (23) zur Führung des zylindrischen Außenumfangs des Einsteckteils (2) vorgesehen sind.

3. Anschlußvorrichtung nach Anspruch 2, dadurch gekennzeichnet, daß jeder Haltevorsprung (14) in Umfangsrichtung durch wenigstens ein in Umfangsrichtung wirksames Anschlagende (16 bzw. 17) begrenzt ist und einer eigenen Ausnehmung (24) des Aufnahmeteils (1) zur Aufnahme des Haltevorsprungs (14) in der Riegelstellung zugeordnet ist, die wenigstens ein in Umfangsrichtung wirksames Gegenanschlagende (26 bzw. 27) zur Beaufschlagung durch das Anschlagende (16 bzw. 17) des Haltevorsprungs (14) bei Drehung des Einsteckteils (2) gegenüber dem Aufnahmeteil (1) aufweist.

4. Anschlußvorrichtung nach Anspruch 2 oder 3, dadurch gekennzeichnet, daß die Umfangsrichtung und/oder in Axialrichtung des Einsteckteils (2) liegende Erstreckung jedes Haltevorsprungs (14) nur geringes Untermaß gegenüber der entsprechenden Abmessung der zugeordneten Ausnehmung (24) des Aufnahmeteils (1) aufweist.

5. Anschlußvorrichtung nach einem der Ansprüche 1 bis 4, dadurch gekennzeichnet, daß jeder Rastvorsprung (28) mit seiner Anschlagfläche (29) Teil eines Randes der zugehörigen Ausnehmung (24) bildet.

6. Anschlußvorrichtung nach einem der Ansprüche 3 bis 5, dadurch gekennzeichnet, daß die Seitenflanken der Führungsrippen (23) die Gegenanschlagenden (26 bzw. 27) der Ausnehmung (24) bilden.

7. Anschlußvorrichtung nach einem der Ansprüche 1 bis 6, dadurch gekennzeichnet, daß wenigstens eine der Ausnehmungen (24) als in Radialrichtung offener Durchbruch ausgebildet ist.

## Claims

1. A union for medical instruments or conduits, such as catheters, probes, hoses etc. in particular for injection, transfusion or infusion, comprising a male half (2) having a duct (13) and having a spigot (11) with at least one radial spigot nosepiece (14) on its circumferential wall (19), and comprising a female half (1) having a duct (8) and having a centre axial aperture provided for receiving said spigot (11) and having at least one locking nosepiece (28) within the motion path of said spigot nosepiece (14) at the coupling movement between said male half (2) and said female half (1) which under elastic deformation allows a penetration of said spigot nosepiece (14) and which comprises at the side being the rear one in penetration direction a stop face (29) constructed and arranged to be acted upon by a rear stop of the penetrated spigot nosepiece (14), whereby the spigot nosepiece (14) is arranged within the area of the front end of said spigot (11) of said male half (2) and in circumferential direction has an angular extent of less than 180°, in particular less than 90°, and whereby the female half (1) comprises between said locking nosepiece (28) and its front end a guide structure (6) circumferentially limiting the insert aperture and at least one axial guide channel (22) for the at least one spigot nosepiece (14) at the inner side of the cylindrical circumferential wall (19) of said guide structure (6), whereby the locking nosepiece (28) is embodied in the rear end of said guide channel (22), characterized in

a) that the male half (2) comprises a spigot (11) of generally round cross-sectional figure,

b) that within the insert aperture of said female half (1) within the axial area of said guide structure (6) a pin (9) is coaxially arranged, whose circumferential surface is — under formation of an annular socket space (20) — at space from the inner side of said circumferential wall (19) of said guide structure (6), and that the pin (9) comprises an inner duct (8),

c) that the circumferential surface of said pin (9) is conically tapered toward said front end in a cone relationship 1 : 16,67 according to a Luer-union and that the inner surface of said duct (13) of said male half (2) is conically flared toward its front end,

d) that the elastic element which allows the penetration of the substantially unelastic spigot nosepiece (14) is the circumferential wall (19) of said guide structure (6), and

e) that each spigot nosepiece (14) comprises a wedge-shaped tapered back face (15) being rear in insertion direction (arrow 14) and that the inclination of said back face (15) corresponds at least approximately to the pitch of a common screw cap for Luer-unions (normally 12°).

2. Union according to claim 1, characterized in that said male half (2) comprises at least two spigot nosepieces (14), that each spigot nosepiece (14) of said male half (2) is diametrically opposite another in pair-like arrangement, and that between the guide channels (22) for said spigot nosepieces (14) guidelands (23) are provided for guiding the cylindrical outer circumference of said male half (2).

3. Union according to claim 2, characterized in that each spigot nosepiece (14) terminates circumferentially by at least one circumferentially acting stop end (16, resp. 17) and is assigned to an own pocket (24) of said female half (1) for receiving said spigot nosepiece (14) in the locked condition, said pocket comprising at least one circumferentially acting counter stop end (26, resp. 27) constructed and arranged to be acted upon by said stop end (16, resp. 17) of said spigot nosepiece (14) at turning said male half (2) in relation to said female half (1).

4. Union according to claim 2 or 3, characterized in that the extension of each spigot nosepiece (14) being in circumferential and/or axial direction of said male half (2) is only somewhat undersized in relation to the respective dimension of the assigned pocket (24) of said female half (1).

5. Union according to one of claims 1 to 4, characterized in that each locking nosepiece (28) with its stop face (29) forms part of an edge of the respective pocket (24).

6. Union according to one of claims 3 to 5, characterized in that the side flanges of the guidelands (23) form the walls (26, resp. 27) of said pocket (24).

7. Union according to one of claims 1 to 6, characterized in that at least one of said pockets (24) is embodied as a radially opening aperture.

## Revendications

1. Raccord pour instruments ou tuyaux médicaux, tels que des cathéters, des sondes, des flexibles, etc., notamment à des fins d'injection, de transfusion ou de perfusion, comprenant une partie emboîtable (2) percée d'un canal (13) de passage traversant et munie d'un tronçon d'emboîtement (11) présentant au moins une saillie radiale de retenue (14) sur sa paroi périphérique (19), ainsi qu'une partie réceptrice (1) percée d'un canal (8) de passage traversant et pourvue d'un orifice médian axial d'emboîtement qui est prévu pour recevoir le tronçon d'emboîtement (11) de la partie emboîtable (2) et qui comporte, sur le trajet de mouvement de la saillie de retenue (14) lors du mouvement d'accouplement entre la partie emboîtable (2) et la partie réceptrice (1), au moins une saillie à déclic (28) autorisant par déformation élastique une pénétration traversante de la saillie de retenue (14) et présentant, à sa face postérieure dans la direction de pénétration traversante, une surface de butée (29) en vue d'une sollicitation par l'intermédiaire d'une butée, postérieure dans la direction de pénétration traversante, de la saillie de retenue insérée (14), ladite saillie de retenue (14) étant disposée au voisinage de l'extrémité antérieure du tronçon d'emboîtement (11) de la partie emboîtable (2) et étant délimitée, dans le sens périphérique, sur une plage angulaire inférieure à 180°, notamment inférieure à 90°, tandis que la partie réceptrice (1) possède, entre la saillie à déclic (28) et son extrémité antérieure, un tronçon de guidage (6) délimitant périphériquement l'orifice d'emboîtement, ainsi qu'au moins un canal axial de guidage (22) pour la saillie de retenue (14) prévue au minimum, à la face interne de la paroi cylindrique périphérique (19) du tronçon de guidage (6), la saillie à déclic (28) étant ménagée dans l'extrémité postérieure du canal de guidage (22), caractérisé par le fait

a) que la partie emboîtable (2) présente un tronçon d'emboîtement (11) de section sensiblement ronde,

b) que l'orifice d'emboîtement de la partie réceptrice (1) loge, dans la région axiale du tronçon de guidage (6), un tenon d'emboîtement (9) disposé coaxialement et dont la surface périphérique se trouve à distance de la face interne de la paroi périphérique (19) du tronçon de guidage (6), en formant un espace intercalaire (20) en forme de canal annulaire, le tenon d'emboîtement (9) comportant un canal interne (8) de passage traversant,

c) que la surface périphérique du tenon d'emboîtement (9) accuse un rétrécissement conique vers l'extrémité antérieure de ce tenon, avec un rapport de conicité de 1 : 16,67, selon une liaison de type « Luer », et la surface interne du canal (13) de passage traversant de la partie emboîtable (2) accuse un élargissement conique vers son extrémité antérieure,

d) que l'élément élastique, autorisant la pénétration traversante de la saillie de retenue (14) sensiblement non douée d'élasticité, est la paroi périphérique (19) du tronçon de guidage (6) et

e) que chaque saillie de retenue (14) présente une face postérieure (15) qui est située à l'arrière dans la direction de l'emboîtement (flèche 4) et possède un biseau cunéiforme, l'inclinaison de cette face postérieure (15) correspondant au moins approximativement au pas de filetage d'un capuchon taraudé classique pour des liaisons du type « Luer » (normalement 12°).

2. Raccord selon la revendication 1, caractérisé par le fait que la partie emboîtable (2) présente au moins deux saillies de retenue (14) ; par le fait que chaque saillie de retenue (14) de la partie emboîtable (2) est diamétralement opposée à une autre saillie de retenue (14), à laquelle elle est associée pour former une paire ; et par le fait que des nervures de guidage (23) sont prévues, entre les canaux (22) de guidage des saillies de retenue (14), en vue de guider le pourtour externe cylindrique de la partie emboîtable (2).

3. Raccord selon la revendication 2, caractérisé par le fait que chaque saillie de retenue (14) est délimitée, dans le sens périphérique, par au moins une extrémité de butée (16 ou 17, respectivement) agissant dans le sens périphérique, et est associée, dans la position verrouillée, à un évidement propre (24) de la partie réceptrice (1) qui est destiné à recevoir la saillie de retenue (14) et qui comporte au moins une extrémité de contre-butée (26 ou 27, respectivement) agissant dans le sens périphérique en vue d'une sollicitation par l'intermédiaire de l'extrémité de butée (16 ou 17, respectivement) de la saillie de retenue (14), lors d'une rotation de la partie emboîtable (2) par rapport à la partie réceptrice (1).

4. Raccord selon la revendication 2 ou 3, caractérisé par le fait que la direction périphérique et/ou l'étendue de chaque saillie de retenue (14), dans le sens axial de la partie emboîtable (2), accuse seulement un surdimensionnement faible par rapport à la dimension correspondante de l'évidement associé (24) de la partie réceptrice (1).

5. Raccord selon l'une des revendications 1 à 4, caractérisé par le fait que chaque saillie à déclic (28) forme, par sa surface de butée (29), une partie d'un bord de l'évidement associé (24).

6. Raccord selon l'une des revendications 3 à 5, caractérisé par le fait que les flancs latéraux des nervures de guidage (23) forment les extrémités de contre-butée (26 ou 27, respectivement) de l'évidement (24).

7. Raccord selon l'une des revendications 1 à 6, caractérisé par le fait qu'au moins l'un des évidements (24) est réalisé sous la forme d'une creusure ouverte dans le sens radial.

**Fig. 2**

**Fig. 1**

**Fig. 3**

**Fig. 4**

0 063 333

**Fig. 5**